# FASCICULE DE BREVET EUROPEEN

(11) **EP 1 937 193 B1**
(45) Date de publication et mention de la délivrance du brevet: **02.12.2009**
(21) Numéro de dépôt: 06820146.6
(22) Date de dépôt: 04.10.2006
(51) Int. Cl.: A61F 5/01, A41D 13/08, A41D 27/10, A61F 13/10, A61F 13/06

(54) **MANCHON ELASTIQUE A GARNITURE VISCOELASTIQUE POUR LA PROTECTION OU LE SOIN DE DOIGTS OU D'ORTEILS**
ELASTISCHE HÜLSE MIT VISKOELASTISCHER AUSKLEIDUNG FÜR FINGER- ODER ZEHENSCHUTZ ODER PFLEGE
ELASTIC SLEEVE WITH VISCOELASTIC LINING FOR FINGER OR TOE PROTECTION OR CARE

(30) Priorité: 20.10.2005 FR 0510670
(43) Date de publication de la demande: 02.07.2008
(73) Titulaire: Millet Innovation, 26270 Loriol sur Drome (FR)
(72) Inventeur: MARTIN, Jean-Luc, F-26270 Loriol sur Drome (FR)
(74) Mandataire: de Roquemaurel, Bruno
(86) Numéro de dépôt international: PCT/FR2006/002231
(87) Numéro de publication internationale: WO 2007/045737

(56) Documents cités:
- EP-B- 1 191 912
- US-A- 2 044 523
- US-A- 3 648 291
- US-A- 5 383 846

## Description

La présente invention concerne le domaine des pansements en forme de manchon pour la protection et le soin des doigts, des orteils et, plus généralement, de membres du corps humain.

Les extrémités des membres du corps humain sont exposées à de nombreuses sollicitations donnant lieu à des blessures ou à des affections qui nécessitent des moyens de protection ou de soin.

Il existe à cet effet diverses structures et formes variées de manchons comportant des matériaux viscoélastiques. On connaît notamment des manchons tubulaires constitués uniquement de polymères viscoélastiques. L'inconvénient de ces manchons est que le tube polymère doit rassembler des propriétés mécaniques d'élasticité, de résistance, de viscoélasticité et d'encombrement réduit qui sont contradictoires et ne sont pas combinés de manière satisfaisante au détriment des effets physiologiques du manchon. On connaît également des manchons tissés comprenant une garniture de matériau viscoélastique. Ces manchons ont pour inconvénient de présenter un fort encombrement, le tissu et la garniture devant avoir une épaisseur importante du fait des mauvaises propriétés mécaniques des matériaux utilisés. En outre, pour résoudre le problème de réalisation industrielle d'un manchon tubulaire en tissu, on fait appel à des tissus tricotés à mailles larges au détriment de l'épaisseur et de la tenue du manchon ainsi que du confort de l'utilisateur.

Le brevet EP 1 191 912 décrit un manchon de protection pour le soin de doigts, orteils ou autres parties du corps, comprenant une pièce de tissu assemblée de manière à former un manchon, et au moins une couche de garniture fixée sur une face de la pièce de tissu.

Ce document décrit plus particulièrement, en relation avec ses figures 1 à 5, un manchon de protection comprenant deux pièces de tissu assemblées par soudage ou couture le long de leurs bords pour former le corps du manchon. La couche de garniture est tout d'abord fixée sur une face d'une des deux pièces de tissu et le manchon est retourné avant d'être utilisé de manière à ce que la couche de garniture soit à l'intérieur du manchon.

Le document EP 1 191 912 décrit également en relation avec ses figures 6 à 8, un mode de réalisation du manchon de protection à partir d'une seule pièce de tissu qui reçoit tout d'abord la couche de garniture et qui est ensuite pliée et assemblée en réalisant par soudure ou couture deux lignes d'assemblage perpendiculaires à la ligne de pliage de la pièce de tissu. On obtient ainsi un manchon fermé à l'une des ses extrémités, l'extrémité fermée correspondant à la ligne de pliage.

Ce procédé de réalisation d'un manchon à partir d'une seule pièce de tissu est avantageux car il simplifie le processus de fabrication. Toutefois, il ne permet pas d'obtenir un manchon ayant ses deux extrémités ouvertes, ni de réaliser collectivement une pluralité de manchons.

De plus, dans le premier ou le second mode de réalisation décrit par EP 1 191 912, les lignes d'assemblage par soudure ou couture forment, malgré le retournement du manchon, des surépaisseurs ou bourrelets d'assemblage, ou encore des zones de matière durcie qui peuvent irriter la peau. Chez certaines personnes comme les diabétiques victimes de neuropathies ou d'artériopathies, de telles irritations provoquent à la longue la formation de lésions.

Ainsi, un objectif de la présente invention est de réaliser un manchon de protection ou de soin de doigt ou d'orteil qui présente des caractéristiques de protection et de confort optimales.

Un autre objectif de la présente invention est de prévoir un procédé de fabrication d'un manchon de protection qui soit simple à mettre en oeuvre et d'un faible prix de revient.

Encore un autre objectif de la présente invention est de prévoir un procédé de fabrication d'un manchon de protection qui permette de réaliser un manchon de protection à partir d'une seule pièce de tissu.

Encore un autre objectif de la présente invention est de prévoir un procédé de fabrication d'un manchon de protection qui permette de réaliser collectivement une pluralité de manchons de protection.

Pour atteindre ces objectifs, une idée de la présente invention est de former un manchon en pliant une pièce de tissu et en réalisant une seule ligne d'assemblage parallèle à la ligne de pliage du tissu. Une autre idée de l'invention est de déposer la couche de garniture du manchon après avoir réalisé les étapes de pliage et d'assemblage de la pièce de tissu, et de déposer cette couche de garniture sur la ligne d'assemblage, de manière qu'un bourrelet d'assemblage soit recouvert en tout ou en partie par la couche de garniture.

Plus particulièrement, la présente invention prévoit un Manchon de protection d'une partie du corps, comprenant une pièce de tissu assemblée de manière à former un manchon, et au moins une couche de garniture fixée sur une face de la pièce de tissu. Selon l'invention, la pièce de tissu ne présente qu'une seule ligne d'assemblage reliant deux de ses bords et s'étendant parallèlement à une ligne de pliage de la pièce de tissu, et la couche de garniture recouvre au moins partiellement la ligne d'assemblage.

Selon un mode de réalisation de l'invention, la couche de garniture est en un gel viscoélastique.

Avantageusement, le gel viscoélastique est auto-adhésif.

Selon un mode de réalisation de l'invention, la pièce de tissu comprend des fibres élastiques.

Selon un mode de réalisation de l'invention, la pièce de tissu comporte des fibres thermoplastiques et la ligne d'assemblage est une ligne de soudure.

Selon un mode de réalisation de l'invention, la ligne d'assemblage est une ligne de couture.

Selon un mode de réalisation de l'invention, la pièce de tissu comprend un mélange de fibres de polyamide et de fibres d'élasthanne.

Selon un mode de réalisation de l'invention, la couche de garniture est fixée par collage sur la pièce de tissu.

L'invention concerne également un procédé de fabrication d'au moins un manchon de protection d'une partie du corps, le manchon comprenant une pièce de tissu assemblée de manière à former un manchon, et au moins une couche de garniture fixée sur une face de la pièce de tissu. Selon l'invention, le procédé comprend des étapes consistant à :
- plier la pièce de tissu et assembler deux bords opposés de la pièce de tissu en suivant une ligne d'assemblage parallèle à une ligne de pliage de la pièce de tissu, pour former un tube ayant deux extrémités ouvertes, et
- fixer au moins une couche de garniture sur une face externe de la pièce de tissu, en recouvrant au moins partiellement la ligne d'assemblage.

Selon un mode de réalisation de l'invention, la pièce de tissu comporte des fibres thermoplastiques et la ligne d'assemblage est réalisée par soudage des bords opposés de la pièce de tissu.

Selon un mode de réalisation de l'invention, la ligne d'assemblage est réalisée par couture des bords opposés de la pièce de tissu.

Selon un mode de réalisation de l'invention, le procédé comprend une étape de découpe transversale du tube à plat pour obtenir une pluralité de manchons.

Selon un mode de réalisation de l'invention, le procédé comprend une étape de retournement du manchon pour disposer la bande de garniture sur la face interne du manchon.

Selon un mode de réalisation de l'invention, le procédé comprend une étape de découpe d'un excédent de tissu entre la ligne d'assemblage et les extrémités des bords opposés de la pièce de tissu.

Selon un mode de réalisation de l'invention, la couche de garniture est fixée sur la pièce de tissu par collage.

Selon un mode de réalisation de l'invention, le procédé comprend des étapes consistant à :
- plier la pièce de tissu en forme de bande sur elle-même le long d'une ligne de pliage de manière à disposer des bords longitudinaux de la bande l'un contre l'autre,
- assembler les bords longitudinaux de la bande en suivant une ligne d'assemblage de manière à obtenir un tube de tissu plat,
- faire tourner le tube de tissu sur lui-même de manière à présenter la ligne d'assemblage sensiblement au milieu d'une face visible du tube à plat,
- enrouler le tube de manière à former un rouleau,
- prévoir une bande de garniture en gel viscoélastique posée à plat sur un support, et
- dérouler le rouleau sur la bande de garniture en collant la bande de garniture sur le tube de manière à recouvrir la ligne d'assemblage.

Ces objets, caractéristiques et avantages ainsi que d'autres de la présente invention seront exposés plus en détail dans la description suivante de modes de réalisation de l'invention, faite à titre non limitatif en relation avec les figures jointes parmi lesquelles :
- les figures 1 à 4 sont des vues en coupe illustrant un procédé de fabrication d'un manchon de protection selon l'invention, représenté à plat ;
- la figure 5 est une vue en coupe du manchon prêt à être utilisé pour la protection d'un doigt ou d'un orteil ;
- la figure 6 est une vue en coupe d'une variante de réalisation du manchon selon l'invention.

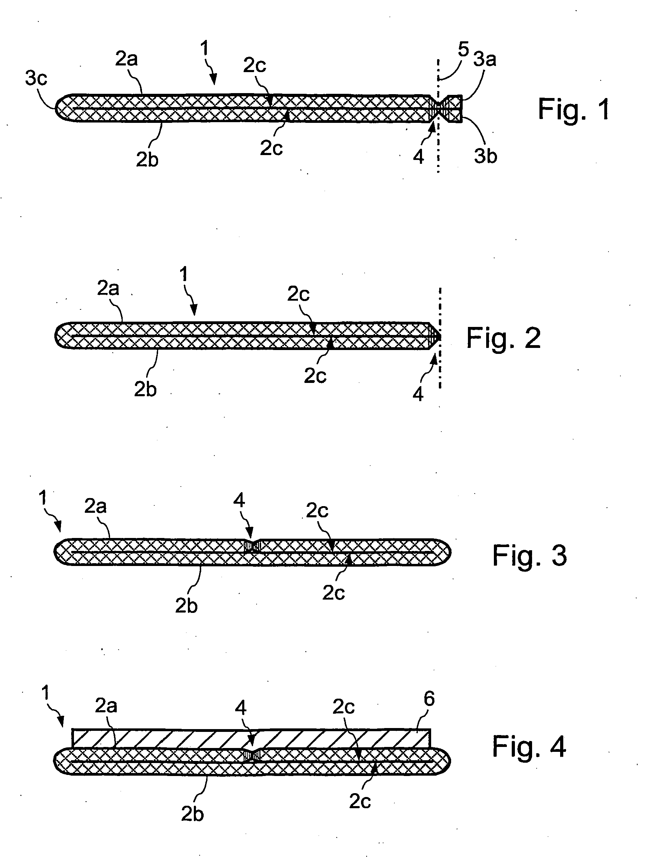

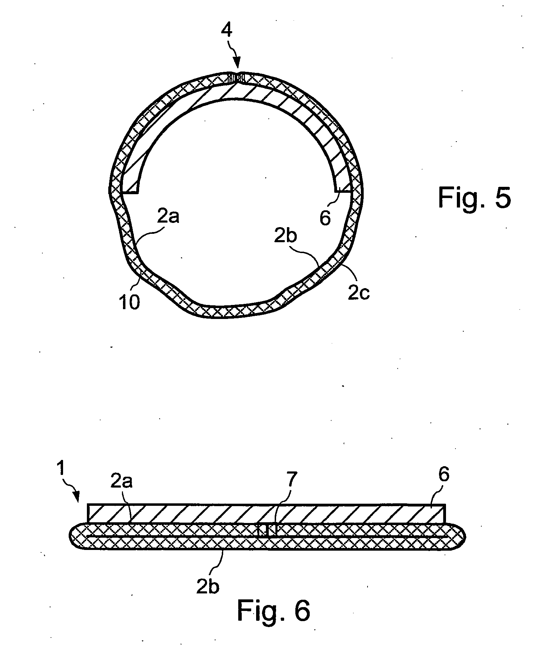

Au cours d'une étape illustrée en figure 1, une bande de tissu rectangulaire est repliée sur elle-même longitudinalement, le long d'une ligne de pliage médiane 3c. Les bords longitudinaux 3a, 3b de la bande de tissu ainsi placés l'un contre l'autre, sont assemblés l'un contre l'autre en réalisant une ligne de soudure 4 parallèle à la ligne de pliage 3c, contrairement au procédé décrit dans le document EP 1 191 912 (figures 6 à 8), prévoyant deux lignes de soudures réalisées perpendiculairement à la ligne de pliage.

On obtient ainsi un tube 1 dont les extrémités sont ouvertes, au lieu d'un manchon ayant une extrémité fermée, et ne présentant qu'une ligne d'assemblage, ici une ligne de soudure.

Sur la figure 1 ainsi qu'au cours d'étapes décrites plus loin et illustrées en figures 2 à 4, le tube 1 est disposé à plat sur un support de travail (non représenté) et présente une forme sensiblement aplatie, qui est représentée schématiquement sur ces figures comme étant parfaitement aplatie. Le tube présente ainsi une face externe supérieure 2a, une face externe inférieure 2b, et deux faces internes 2c se trouvant en regard.

La réalisation de la ligne de soudure 4 est effectuée de façon classique à l'aide d'une machine de thermosoudage ou de soudure aux ultrasons produisant un échauffement localisé entraînant une fusion du tissu. A cet effet, la bande de tissu comporte des fibres thermoplastiques. Elle comprend également, de préférence, des fibres élastiques conférant au tube l'élasticité désirée.

Au cours de l'étape illustrée sur la figure 2, l'excédent de tissu au niveau des bords assemblés est retiré par découpe le long d'une ligne de découpe 5. De préférence, la découpe des bords du tissu est effectuée au moment de la soudure par l'outil de soudure qui fait fondre le tissu. La zone de soudure forme alors en coupe une sorte de bec de matière durcie.

Au cours de l'étape illustrée sur la figure 3, le tube 1 est tourné sur lui-même de manière à amener la ligne de soudure 4 sur la face externe supérieure 2a du tube 1, qui est toujours disposé à plat. De préférence, la ligne de soudure est placée sensiblement au milieu de la face externe supérieure 2a. Durant le mouvement de rotation du tube sur lui-même, les bords assemblés 3a, 3b s'écartent pour former une liaison bord à bord du tissu. De cette manière, l'assemblage des bords de la bande de tissu ne forme aucune surépaisseur susceptible de gêner l'utilisateur ou de provoquer des lésions.

Au cours d'une étape suivante, illustrée par la figure 4, une garniture 6 est fixée sur le tube 1, de manière à recouvrir la ligne de soudure 4, au moins partiellement et de préférence en totalité. Avantageusement, la garniture 6 est une bande constituée par un gel de silicone viscoélastique, d'une épaisseur uniforme de quelques dixièmes de millimètres à quelques millimètres, et de préférence de l'ordre de 1 mm. La garniture 6 est par exemple fabriquée à l'épaisseur désirée et découpée en bandes ayant la même largeur que le tube 1 à plat, par exemple au moyen de couteaux rotatifs.

La garniture est assemblée sur le tube 1 par collage sur la face externe supérieure 2a du tube. Par exemple, le tube de tissu à plat 1 est déroulé et mis en contact avec une bande de gel de silicone sensiblement de même largeur, en disposant de la colle entre la face externe supérieure 2a et la bande de gel. Dans une variante, la bande de gel de silicone et/ou la face externe supérieure du tube sont préencollées.

Avant la fixation de la bande gel de silicone, le tube 1 peut être enroulé de manière à former un rouleau, tandis que la bande de gel de silicone posée à plat sur un support. Le tube est ensuite déroulé sur la bande de gel de silicone en encollant préalablement l'une et ou l'autre des faces en regard du tube et de la bande de gel de silicone. Le tube 1 peut ainsi être fabriqué quasiment en continu, ce qui permet de réduire les chutes de tissu.

Le tube 1 selon l'invention, prévu ici d'une longueur nettement supérieure à la longueur usuelle d'un manchon de protection, est ensuite sectionné de manière à obtenir des manchons présentant une longueur désirée.

Comme illustré sur la figure 5, les manchons découpés 10 obtenus à la suite du découpage du tube 1, sont retournés au moment de leur utilisation, de manière à ce que la garniture 6 se trouve à l'intérieur du manchon. Cependant, ce retournement est facultatif étant donné que le manchon selon l'invention peut également être utilisé avec la couche de gel viscoélastique disposée extérieurement, par exemple pour protéger un doigt adjacent à celui recouvert par le manchon.

Le manchon 10 ainsi obtenu ne présente aucune couture ou ligne d'assemblage apparente du côté où est fixée la garniture 6. La garniture 6 collée sur le tube de tissu de chaque côté de la ligne de soudure 4 et sur celle-ci renforce la ligne de soudure et limite ainsi le risque de déchirement.

En outre, lorsque le mode d'assemblage des bords du tissu entraîne la formation d'un bourrelet, celui-ci est avantageusement recouvert par la couche de gel viscoélastique qui se déforme lors de sa fixation pour ne former aucune surépaisseur du côté de sa face destinée à venir en contact avec la peau.

La bande de tissu constituant le tube de tissu 1 comprend de préférence un mélange de fibres élastiques constituées d'élasthanne, et de fibres thermoplastiques constituées de polyamide. A titre d'exemple, la bande de tissu comprend 70% de fibres de polyamide et 30% de fibres d'élasthanne. Avantageusement, le grammage de la bande de tissu est inférieur à 250 g/cm². De préférence, le tissu choisi est indémaillable pour éviter tout effilochage à la suite de la découpe du tube en manchons.

Le gel de silicone formant la garniture 6 présente de préférence des propriétés de viscoélasticité similaires aux propriétés mécaniques de certains tissus cutanés humains. Des compositions de mélanges de gels de silicone présentant de telles propriétés ont été décrites dans le brevet FR 2 712 487 auquel on se référera pour des détails de formulation et d'élaboration. Ces compositions ont la particularité de reproduire les propriétés mécaniques du capiton plantaire naturel et de présenter notamment des valeurs similaires de modules en compression et en torsion. Des gels silicone ayant de telles formulations sont commercialisés par la demanderesse sous l'appellation EPITHELIUM 26^{®}. D'autres formulations, commercialisées par la demanderesse sous l'appellation EPITHELIUM 27^{®} et EPIHELIUM 27+^{®}, permettent en outre d'obtenir, parfois au détriment de la similarité des modules mécaniques en compression et en torsion, des propriétés d'adhérence intrinsèque du gel silicone. Ces gels présentent des qualités de contact exceptionnelles et constituent des répartiteurs de charge excellents susceptibles de donner des résultats remarquables en confort et en prévention, ainsi qu'en traitement de certaines affections, en particulier des hyperkératoses. En pratique, la conformité de ces gels peut être vérifiée en contrôlant les modules en compression et en torsion dont les valeurs nominales sont de l'ordre de 4.103 N/m2 et de 4.103 N/m² à 15.103 N/m², respectivement. Une tolérance de 50% par rapport aux valeurs limites de ces modules peut être acceptée sans perdre les avantages de ces gels pour l'application médicale ou para-médicale visée ici.

Ainsi, selon un mode de réalisation préféré de la présente invention, le gel formant la garniture 6 du tube 1 est un gel silicone viscoélastique dont les propriétés mécaniques sont de l'ordre de celles du capiton plantaire humain, le gel ayant notamment des modules en compression et en torsion de l'ordre de la moitié au double des modules correspondants du capiton plantaire. Il s'agit par exemple du gel silicone susmentionné commercialisé sous l'appellation EPITHELIUM 26^{®}.

Dans une variante de réalisation, le gel silicone présente, outre des propriétés mécaniques similaires au capiton plantaire humain, la propriété d'être adhérent intrinsèquement par simple effet de contact avec la peau, le gel pouvant se décoller et se recoller quasi indéfiniment. Il s'agit par exemple du gel silicone commercialisé sous l'appellation EPITHELIUM 27 OU EPITHELIUM 27+.

Dans une variante de réalisation du manchon selon l'invention, illustrée par la figure 6, les deux bords 3a, 3b de la bande de tissu sont assemblés bord à bord par une couture à plat 7 qui ne forme aucune surépaisseur. Comme le fil de couture est recouvert par la bande de gel de silicone 6, il ne risque pas d'entraîner des irritations de la peau.

Il apparaîtra clairement à l'homme de l'art que le dispositif selon la présente invention est susceptible de diverses autres variantes de réalisation et applications. Ainsi, l'invention n'est pas limitée à la fabrication collective de manchons à partir d'une bande de tissu. Un tel manchon peut également être obtenu à partir d'une pièce de tissu rectangulaire. Il n'est pas non plus indispensable de découper les bords excédentaires de la pièce de tissu à la suite de leur fixation. En effet, la soudure peut être effectuée au raz des bords 3a, 3b. L'usage d'un tissu élastique n'est pas non plus nécessaire. Cette caractéristique est simplement préférable pour des raisons de confort d'utilisation du manchon.

De plus, bien que l'on indique dans la présente demande que le manchon selon l'invention ne présente qu'une ligne d'assemblage, par contraste avec le procédé décrit dans le document EP 1 191 912, il va de soi que cette ligne d'assemblage, notamment si elle est réalisée par couture, peut comprendre plusieurs lignes de couture parallèles et rapprochées formant ensemble une ligne d'assemblage unique au sens de l'invention.

De même, d'autres moyens de fixation peuvent être envisagés pour fixer la couche de gel viscoélastique.

## Revendications

1. Manchon(10) de protection d'une partie du corps, comprenant une pièce de tissu (1) assemblée de manière à former un manchon, et au moins une couche de garniture (6) fixée sur une face (2a) de la pièce de tissu,
**caractérisé en ce que** la pièce de tissu ne présente qu'une seule ligne d'assemblage (4, 7) reliant deux de ses bords (3a, 3b) et s'étendant parallèlement à une ligne de pliage (3c) de la pièce de tissu, et **en ce que** la couche de garniture (6) recouvre au moins partiellement la ligne d'assemblage (4, 7).

2. Manchon selon la revendication 1, dans lequel la couche de garniture (6) est en un gel viscoélastique.

3. Manchon selon la revendication 1 ou 2, dans lequel la pièce de tissu (1) comprend des fibres élastiques.

4. Manchon selon l'une des revendications 1 à 3,
dans lequel la pièce de tissu (1) comporte des fibres thermoplastiques et la ligne d'assemblage est une ligne de soudure (4).

5. Manchon selon l'une des revendications 1 à 3,
dans lequel la ligne d'assemblage est une ligne de couture (7).

6. Manchon selon l'une des revendications 1 à 5,
dans lequel la pièce de tissu (1) comprend un mélange de fibres de polyamide et de fibres d'élasthanne.

7. Manchon selon l'une des revendications 1 à 6,
dans lequel la couche de garniture (6) est fixée par collage sur la pièce de tissu (1).

8. Manchon selon l'une des revendications 1 à 7,
dans lequel le gel viscoélastique (6) est auto-adhésif.

9. Procédé de fabrication d'au moins un manchon (10) de protection d'une partie du corps, le manchon comprenant une pièce de tissu (1) assemblée de manière à former un manchon, et au moins une couche de garniture (6) fixée sur une face(2a) de la pièce de tissu,
**caractérisé en ce qu'**il comprend des étapes consistant à :
- plier la pièce de tissu(1) et assembler deux bords opposés (3a, 3b) de la pièce de tissu en suivant une ligne d'assemblage (4, 7) parallèle à une ligne de pliage (3c) de la pièce de tissu, pour former un tube ayant deux extrémités ouvertes, et
- fixer au moins une couche de garniture (6) sur une face externe (2a) de la pièce de tissu (1), en recouvrant au moins partiellement la ligne d'assemblage (4, 7).

10. Procédé selon la revendication 9, dans lequel la pièce de tissu comporte des fibres thermoplastiques et la ligne d'assemblage (4) est réalisée par soudage des bords opposés (3a, 3b) de la pièce de tissu.

11. Procédé selon la revendication 9, dans lequel la ligne d'assemblage (7) est réalisée par couture des bords opposés (3a, 3b) de la pièce de tissu.

12. Procédé selon l'une des revendications 9 à 11, comprenant une étape de découpe transversale du tube à plat (1) pour obtenir une pluralité de manchons (10).

13. Procédé selon l'une des revendications 9 à 12, comprenant une étape de retournement du manchon (10) pour disposer la bande de garniture sur la face interne du manchon.

14. Procédé selon l'une des revendications 9 à 13,
dans lequel la couche de garniture est en un gel viscoélastique (6).

15. Procédé selon l'une des revendications 9 à 14, comprenant une étape de découpe d'un excédent de tissu entre la ligne d'assemblage (4) et les extrémités des bords opposés (3a, 3b) de la pièce de tissu (1).

16. Procédé selon l'une des revendications 9 à 15,
dans lequel la couche de garniture (6) est fixée sur la pièce de tissu (1) par collage.

17. Procédé selon l'une des revendications 9 à 16, comprenant des étapes consistant à :
- plier la pièce de tissu (1) en forme de bande sur elle-même le long d'une ligne de pliage (3c) de manière à disposer des bords longitudinaux (3a, 3b) de la bande l'un contre l'autre,
- assembler les bords longitudinaux (3a, 3b) de la bande en suivant une ligne d'assemblage (4, 7) de manière à obtenir un tube de tissu plat,
- faire tourner le tube de tissu sur lui-même de manière à présenter la ligne d'assemblage (4, 7) sensiblement au milieu d'une face visible du tube à plat,
- enrouler le tube de manière à former un rouleau,
- prévoir une bande de garniture (6) en gel viscoélastique posée à plat sur un support, et
- dérouler le rouleau sur la bande de garniture en collant la bande de garniture sur le tube de manière à recouvrir la ligne d'assemblage.

## Claims

1. A sleeve (10) for protecting a part of the body, comprising a piece of fabric (1) assembled so as to form a sleeve, and at least one lining layer (6) fixed onto a face (2a) of the piece of fabric,
**characterized in that** the piece of fabric has only one assembly seam (4, 7) linking two of its edges (3a, 3b) and extending in parallel to a fold line (3c) of the piece of fabric, and **in that** the lining layer (6) covers at least partially the assembly seam (4, 7).

2. Sleeve according to claim 1, wherein the lining layer (6) is made of a viscoelastic gel.

3. Sleeve according to claim 1 or 2, wherein the piece of fabric (1) comprises elastic fibers.

4. Sleeve according to one of claims 1 to 3, wherein the piece of fabric (1) comprises thermoplastic fibers and the assembly seam is a weld seam (4).

5. Sleeve according to one of claims 1 to 3, wherein the assembly seam is a stitch seam (7).

6. Sleeve according to one of claims 1 to 5, wherein the piece of fabric (1) comprises a combination of polyamide fibers and elastane fibers.

7. Sleeve according to one of claims 1 to 6, wherein the lining layer (6) is fixed by being stuck onto the piece of fabric (1).

8. Sleeve according to one of claims 1 to 7, wherein the viscoelastic gel (6) is self-adhesive.

9. A method for manufacturing at least one sleeve (10) for protecting a part of the body, the sleeve comprising a piece of fabric (1) assembled so as to form a sleeve, and at least one lining layer (6) fixed onto a face (2a) of the piece of fabric, **characterized in that** it comprises steps of:
- folding the piece of fabric (1) and assembling two opposite edges (3a, 3b) of the piece of fabric along an assembly seam (4, 7) parallel to a fold line (3c) of the piece of fabric, to form a tube having two open ends, and
- fixing at least one lining layer (6) onto an external face (2a) of the piece of fabric (1), covering at least partially the assembly seam (4, 7).

10. Method according to claim 9, wherein the piece of fabric comprises thermoplastic fibers and the assembly seam (4) is produced by welding the opposite edges (3a, 3b) of the piece of fabric.

11. Method according to claim 9, wherein the assembly seam (7) is produced by stitching the opposite edges (3a, 3b) of the piece of fabric.

12. Method according to one of claims 9 to 11, comprising a step of cross cutting the tube (1) when flat to obtain a plurality of sleeves (10).

13. Method according to one of claims 9 to 12, comprising a step of turning the sleeve (10) inside out to arrange the lining strip on the internal face of the sleeve.

14. Method according to one of claims 9 to 13, wherein the lining layer is made of a viscoelastic gel (6).

15. Method according to one of claims 9 to 14, comprising a step of cutting an excess of fabric between the assembly seam (4) and the ends of the opposite edges (3a, 3b) of the piece of fabric (1).

16. Method according to one of claims 9 to 15, wherein the lining layer (6) is fixed by being stuck onto the piece of fabric (1).

17. Method according to one of claims 9 to 16, comprising steps of:
- folding the piece of fabric (1) over in the form of a strip along a fold line (3c) so as to arrange longitudinal edges (3a, 3b) of the strip against each other,
- assembling the longitudinal edges (3a, 3b) of the strip along an assembly seam (4, 7) so as to obtain a flat tube of fabric,
- turning the tube of fabric about itself so as to present the assembly seam (4, 7) substantially in the middle of a visible face of the flat tube,
- winding the tube so as to form a roll,
- providing a lining strip (6) made of a viscoelastic gel laid down flat on a medium, and
- unwinding the roll onto the lining strip while sticking the lining strip onto the tube so as to cover the assembly seam.

## Patentansprüche

1. Muffe (10) zum Schützen eines Teils des Körpers, umfassend ein Gewebeteil (1), das derart zusammengesetzt ist, dass es eine Muffe bildet, und zumindest eine Verkleidungsschicht (6), die auf einer Seite (2a) des Gewebeteils befestigt ist,
**dadurch gekennzeichnet, dass** das Gewebeteil nur eine einzige Zusammensetzlinie (4, 7) aufweist, die zwei seiner Ränder (3a, 3b) miteinander verbindet und sich parallel zu einer Faltlinie (3c) des Gewebeteils erstreckt, und **dadurch** dass die Verkleidungsschicht (6) zumindest teilweise die Zusammensetzlinie (4, 7) überdeckt.

2. Muffe nach Anspruch 1, in der die Verkleidungsschicht (6) aus einem viskoelastischen Gel ist.

3. Muffe nach Anspruch 1 oder 2, in der das Gewebeteil (1) elastische Fasern umfasst.

4. Muffe nach einem der Ansprüche 1 bis 3, in der das Gewebeteil (1) thermoplastische Fasern aufweist und die Zusammensetzlinie eine Schweißlinie (4) ist.

5. Muffe nach einem der Ansprüche 1 bis 3, in der die Zusammensetzlinie eine Nahtlinie (7) ist.

6. Muffe nach einem der Ansprüche 1 bis 5, in der das Gewebeteil (1) eine Mischung aus Polyamidfasern und aus Elastanfasern umfasst.

7. Muffe nach einem der Ansprüche 1 bis 6, in der die Verkleidungsschicht (6) durch Kleben am Gewebeteil (1) befestigt ist.

8. Muffe nach einem der Ansprüche 1 bis 7, in der das viskoelastische Gel (6) selbsthaftend ist.

9. Verfahren zur Fertigung von zumindest einer Muffe (10) für den Schutz eines Teils des Körpers, wobei die Muffe ein Gewebeteil (1), das derart zusammengesetzt ist, dass es eine Muffe bildet, und zumindest eine Verkleidungsschicht (6), die an einer Seite (2a) des Gewebeteils befestigt ist, umfasst,
**dadurch gekennzeichnet, dass** es Schritte umfasst, die folgendes beinhalten:
- das Gewebeteil (1) falten und zwei gegenüber liegende Ränder (3a, 3b) des Gewebeteils an einer Zusammensetzlinie (4, 7), die parallel zu einer Faltlinie (3c) des Gewebeteils ist, entlang zusammensetzen, um einen Schlauch mit zwei offenen Enden zu bilden, und
- zumindest eine Verkleidungsschicht (6) auf einer externen Seite (2a) des Gewebeteils (1) befestigen,
wobei die Zusammensetzlinie (4, 7) zumindest teilweise überdeckt wird.

10. Verfahren nach Anspruch 9, in der das Gewebeteil thermoplastische Fasern aufweist und die Zusammensetzlinie (4) durch Schweißen der gegenüber liegenden Ränder (3a, 3b) des Gewebeteils realisiert wird.

11. Verfahren nach Anspruch 9, in der die Zusammensetzlinie (7) durch Nähen der gegenüber liegenden Ränder (3a, 3b) des Gewebeteils realisiert wird.

12. Verfahren nach einem der Ansprüche 9 bis 11, umfassend einen Schritt des transversalen Schneidens des flachliegenden Schlauches (1), um eine Vielzahl von Muffen (10) zu erhalten.

13. Verfahren nach einem der Ansprüche 9 bis 12, umfassend einen Schritt des Wendens der Muffe (10), um den Verkleidungsstreifen auf der internen Seite der Muffe anzuordnen.

14. Verfahren nach einem der Ansprüche 9 bis 13, in der die Verkleidungsschicht aus einem viskoelastischen Gel (6) ist.

15. Verfahren nach einem der Ansprüche 9 bis 14, umfassend einen Schritt des Schneidens eines Gewebeüberschusses zwischen der Zusammensetzlinie (4) und den Enden der gegenüber liegenden Ränder (3a, 3b) des Gewebeteils (1).

16. Verfahren nach einem der Ansprüche 9 bis 15, in der die Verkleidungsschicht (6) durch Kleben auf dem Gewebeteil (1) befestigt ist.

17. Verfahren nach einem der Ansprüche 9 bis 16, umfassend Schritte, die folgendes beinhalten:
- das Gewebeteil (1) in Form eines Streifens derart an einer Faltlinie (3c) entlang über sich selbst falten, dass longitudinale Ränder (3a, 3b) des Streifens aneinander angeordnet werden,
- die longitudinalen Ränder (3a, 3b) des Streifens derart an einer Zusammensetzlinie (4, 7) entlang zusammen setzen, dass ein Flachgewebeschlauch erhalten wird,
- den Gewebeschlauch derart um sich selbst drehen, dass die Zusammensetzlinie (4, 7) im Wesentlichen in der Mitte einer sichtbaren Seite des flachliegenden Schlauches angesetzt wird,
- den Schlauch derart wickeln, dass eine Rolle gebildet wird,
- einen Verkleidungsstreifen (6) aus viskoelastischem Gel, der flach auf einen Träger gelegt ist, vorsehen, und
- die Rolle auf dem Verkleidungsstreifen abwickeln, wobei der Verkleidungsstreifen derart auf den Schlauch geklebt wird, dass die Zusammensetzlinie überdeckt wird.
